**Europäisches Patentamt**

**European Patent Office**

**Office européen des brevets**

(11) Numéro de publication : **0 327 459 B1**

(12) **FASCICULE DE BREVET EUROPEEN**

(45) Date de publication du fascicule du brevet :
16.09.92 Bulletin 92/38

(51) Int. Cl.$^5$ : **A61M 25/02, A61B 8/00**

(21) Numéro de dépôt : **89400291.4**

(22) Date de dépôt : **02.02.89**

(54) **Support de sonde échograhique notamment de sonde échocardiograhique.**

(30) Priorité : **05.02.88 FR 8801378**

(43) Date de publication de la demande :
**09.08.89 Bulletin 89/32**

(45) Mention de la délivrance du brevet :
**16.09.92 Bulletin 92/38**

(84) Etats contractants désignés :
**AT BE CH DE ES FR GB GR IT LI LU NL SE**

(56) Documents cités :
**FR-A- 2 502 932**
**FR-A- 2 606 625**
**US-A- 3 893 449**
**US-A- 4 483 344**

(73) Titulaire : **Puy, Philippe**
**7, allée Claire Fontaine**
**F-30400 Villeneuve-les-Avignon (FR)**
Titulaire : **Dreyer, Alain**
**Mas Desgrives**
**D-84470 Chateauneuf-de-Gadagne (FR)**

(72) Inventeur : **Puy, Philippe**
**7, allée Claire Fontaine**
**F-30400 Villeneuve-les-Avignon (FR)**
Inventeur : **Dreyer, Alain**
**Mas Desgrives**
**D-84470 Chateauneuf-de-Gadagne (FR)**

(74) Mandataire : **Schrimpf, Robert et al**
**Cabinet Regimbeau 26, Avenue Kléber**
**F-75116 Paris (FR)**

EP 0 327 459 B1

## Description

La présente invention concerne les supports de sondes échographiques, notamment de sondes échocardiographiques.

De tels supports sont décrits notamment dans les brevets français 2502 932 et 2606 625 et dans les brevets US 3 893 449 et 4 483 344.

Ces supports servent à maintenir une sonde contre un point du corps du patient, tout en permettant un certain réglage de la position angulaire de l'axe de la sonde par rapport à la surface avec laquelle elle se trouve en contact.

Ce positionnement angulaire a pour but d'orienter la sonde en direction de l'organe à examiner, la sonde ne pouvant pas toujours être placée au droit de celui-ci.

Ainsi, dans le cas d'une échocardiographie, le praticien doit tout d'abord rechercher la fenêtre intercostale sur le thorax du patient, appliquer et appuyer la sonde à cet endroit, puis l'orienter de manière à viser la région du coeur à examiner en recherchant le meilleur angle de prise de vue.

Les brevets précités décrivent des supports de sonde qui comprennent

– un socle présentant une surface de contact apte à être appliquée contre le corps du patient à l'endroit à examiner,

– un porte-sonde, recevant de manière amovible la sonde et pourvu de moyens de sollicitation axiale de la sonde contre le corps du patient,

– des moyens de montage du porte-sonde sur le socle avec possibilité d'orientation du porte-sonde par pivotement, et

– des moyens de blocage en position du porte-sonde.

Il est souhaitable, dans la pratique, que le pivotement du porte-sonde se fasse autour d'un point situé à proximité immédiate de la surface de contact, ce que préconisent le brevet français 2502 932 et son équivalent US 4 483 344.

Cependant, le support décrit dans ces deux brevets n'autorise qu'un faible débattement angulaire du porte-sonde par rapport à la normale au plan de contact, ce qui peut être gênant dans un certain nombre de situations. Cet inconvénient est compensé dans une certaine mesure par le fait que le porte-sonde est monté sur un équipage mobile en translation selon deux directions perpendiculaires, afin de pouvoir modifier le point de contact sans changer la position du socle, qui est fixé sur le corps du patient par des sangles, mais il en résulte que la structure mécanique de ce support de sonde est complexe, et ne permet pas un changement rapide de la sonde en raison du démontage nécessaire des différentes pièces du porte-sonde.

L'un des buts de la présente invention est de proposer un support de sonde du type précité qui pallie ces inconvénients en assurant un très large débattement angulaire par rapport à la normale au plan de contact, les rotations étant effectuées autour d'un point fixe le plus rapproché possible du corps du patient.

A cet effet, selon la présente invention :

– les moyens de montage du porte-sonde sur le socle comprennent deux arceaux semi-circulaires montés à pivotement sur le socle autour d'axes respectifs perpendiculaires entre eux et s'étendant dans un plan commun qui est parallèle au plan du socle et qui est situé à proximité de la surface de contact, ces arceaux présentant chacun une fente allongée s'étendant sur la plus grande partie de leur longueur, et

– le porte-sonde est placé dans les deux fentes des arceaux, à l'intersection de celles-ci.

Selon d'autres caractéristiques avantageuses :

– le porte-sonde est de forme sensiblement cylindrique, de manière a autoriser une rotation axiale de la sonde sur elle-même, sans déplacement du socle ni des arceaux ;

– les moyens de blocage comprennent un écrou disposé sur une partie filetée du porte-sonde en saillie par rapport aux arceaux, le serrage de cet écrou assurant en un seul mouvement le blocage et l'immobilisation en position de l'ensemble arceaux/porte-sonde ;

– le débattement angulaire de la sonde est possible à l'intérieur d'un cône de 90° d'angle au sommet et d'axe perpendiculaire à la surface de contact.

D'autres caractéristiques et avantages de la présente invention apparaîtront à la lecture de la description détaillée ci-dessous, faite en référence aux dessins annexés, sur lesquels :

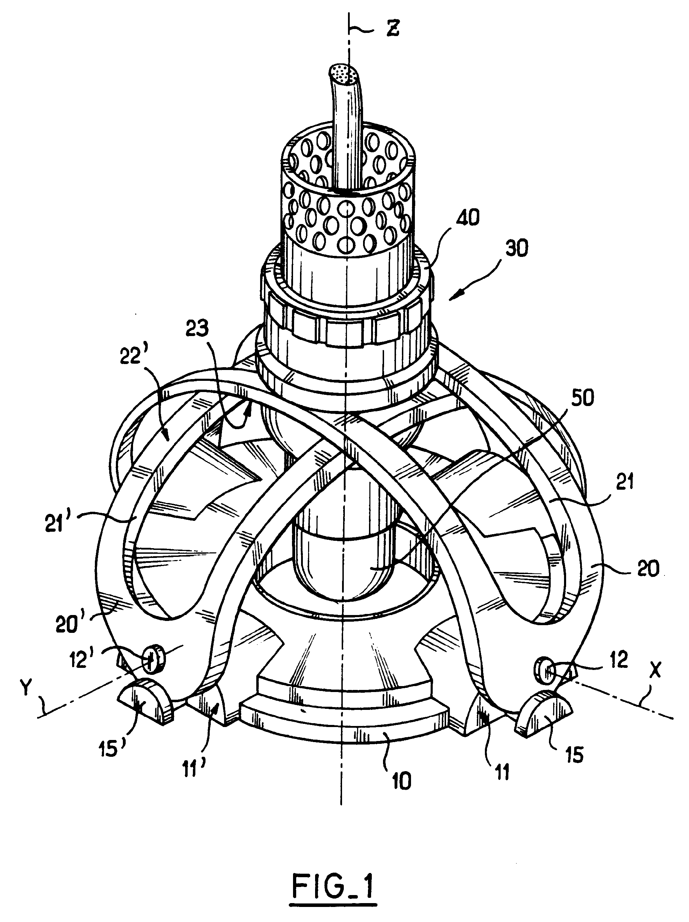

– la figure 1 est une vue perspective du support de sonde selon l'invention, pourvu d'une sonde échographique,

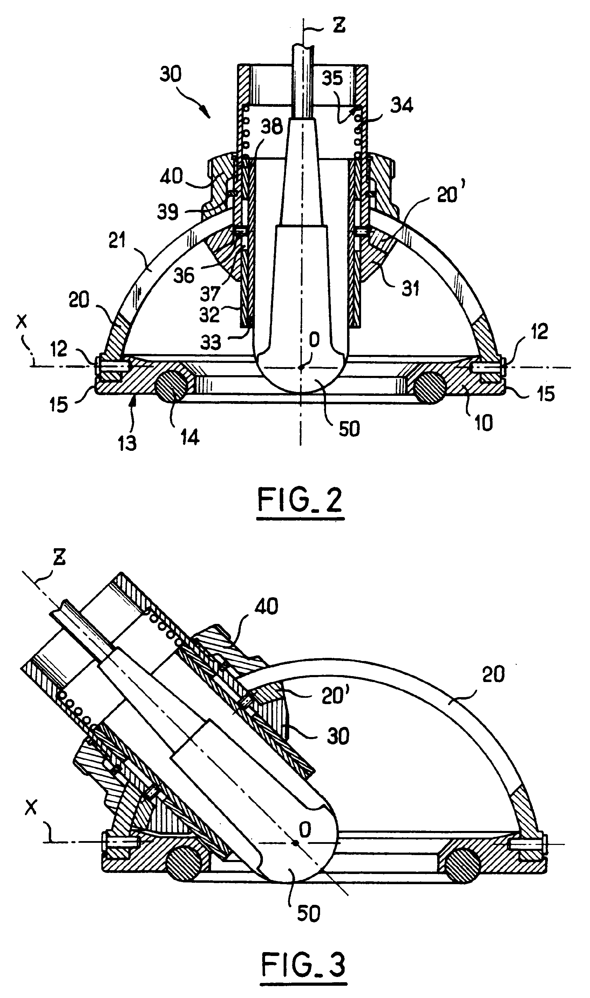

– les figures 2 e t 3 sont des coupes dans le plan XOZ de l'ensemble de la figure 1, respectivement avec une sonde orientée en direction normale par rapport au corps du patient et orientée avec inclinaison maximale.

Sur les figures, on a représenté le support de sonde, constitué des éléments référencés 10 à 40, servant au montage d'une sonde échographique 50.

Le support de sonde comprend un socle 10, de forme sensiblement circulaire, avec quatre méplats 11,11′ disposés a angle droit, sur lesquels sont placés des axes 12,12′ orientés selon des axes orthogonaux X et Y.

Ces axes, qui se coupent au point O situé au centre du socle, s'étendent dans un plan commun parallèle à la surface de contact 13 par laquelle le socle vient s'appliquer contre le corps du patient avec interposition d'un bourrelet 14, ce plan commun étant situé à faible distance de la surface 13.

Les axes 12,12′ reçoivent deux arceaux mobiles 20 et 20′ de configuration similaire, l'arceau 20 étant de dimension légèrement supérieure de manière à chevaucher l'arceau 20′. Ces arceaux s'articulent autour des axes respectifs 12 et 12′, un rebord de protection 15,15′ étant prévu sur le socle pour éviter que le frottement des arceaux contre le corps du patient ne vienne gêner leur rotation.

Ces arceaux sont pourvus, sur la plus grande partie de leur longueur, d'une fente allongée 21,21′, un porte-sonde 30 étant placé dans ces deux fentes à leur intersection.

La surface supérieure 22′ de l'arceau inférieur 21′ et la surface inférieure 23 de l'arceau supérieur 20 sont de préférence des surfaces hémisphériques identiques, afin de permettre un déplacement relatif sans jeu des deux arceaux l'un sur l'autre.

Le porte-sonde 30 est muni de moyens quelconques appropriés pour le montage de la sonde dans le porte-sonde. Ces moyens comprennent par exemple un corps 31 recevant un fourreau 32 cylindrique, lui-même pourvu intérieurement d'un manchon 33 permettant de serrer en position la sonde 50. Le fourreau 32, et donc la sonde 50, est sollicité élastiquement vers le bas par un ressort 34 inséré à l'intérieur du porte-sonde entre le fourreau 32 et un épaulement interne 35 du corps 31 du porte-sonde.

Par ailleurs, on immobilise le fourreau 32 en rotation (rotation sur lui-même autour de l'axe Z de la sonde) au moyen de pions 36 solidaires du corps de porte-sonde 31 et coopérant avec une gorge 37, parallèle à l'axe Z, pratiquée à la périphérie du fourreau 32.

Le corps 31 présente en outre à sa surface extérieure une partie filetée 38, en saillie par rapport aux arceaux 20,20′ et recevant un écrou moleté 40 permettant de bloquer fermement en position l'ensemble une fois atteinte la position angulaire recherchée. Lorsque l'écrou est desserré, le corps du porte-sonde est retenu en place sur les arceaux au moyen d'un clip 39.

Grâce à cette configuration on assure le blocage en position de l'ensemble sans aucun démontage du porte-sonde et avec un unique mouvement de serrage.

Quant à la sonde, elle est fixée dans l'appareil en la passant par le haut et elle est maintenue en place grâce au manchon, ce qui permet de la retirer en gardant les moyens de blocage en position serrée, c'est à dire sans aucun démontage ni déréglage du porte-sonde.

En outre, le système de maintien de la sonde par manchon permet d'utiliser directement des sondes de diamètres différents sans aucune pièce d'adaptation intercalaire, le diamètre maximum étant simplement prévu pour pouvoir recevoir les sondes les plus grosses.

Enfin, le système est très aisé à manipuler d'une seule main par le praticien, celui-ci se contentant de desserrer légèrement l'écrou 40, rechercher l'angle d'examen optimal et resserrer l'écrou 40 une fois cette position atteinte. Pendant la phase d'ajustement angulaire, la sonde pivotera autour du point O, c'est à dire que son centre de rotation sera pratiquement confondu avec le point de contact sonde/corps du patient.

Enfin, pendant l'examen il sera facile, sans démonter le socle ni le porte-sonde, de reculer légèrement la sonde pour placer du gel de contact à l'extrémité de celle-ci, la sonde étant ensuite replacée exactement à la position qu'elle occupait auparavant.

## Revendications

1. Un support de sonde échographique, notamment de sonde échocardiographique, comprenant :
   – un socle (10) présentant une surface de contact apte à être appliquée contre le corps du patient à l'endroit à examiner,
   – un porte-sonde (30), recevant de manière amovible la sonde (50) et pourvu de moyens de sollicitation axiale de la sonde contre le corps du patient,
   – des moyens de montage du porte-sonde sur le socle avec possibilité d'orientation du porte-sonde par pivotement autour d'un point situé à proximité de la surface de contact, et
   – des moyens (40) de blocage en position du porte-sonde.
   caractérisé en ce que,
   – les moyens de montage du porte-sonde sur le socle comprennent deux arceaux semi-circulaires (20,20′) montés à pivotement autour d'axes respectifs (X,Y) perpendiculaires entre eux et s'étendant dans un plan commun, qui est parallèle au plan du socle et qui est situé à proximité immédiate de la surface de contact en sorte que la sonde pivote autour d'un centre de rotation pratiquement confondu avec le point de contact de la sonde avec le patient, ces arceaux présentant chacun une fente (21,21′) allongée s'étendant sur la plus grande partie de leur longueur, et
   – le porte-sonde étant placé dans les deux fentes des arceaux, à l'intersection de celles-ci.

2. Le support de sonde de la revendication 1, dans lequel le porte-sonde comprend un fourreau (32) ouvert vers le haut de manière à recevoir la sonde sans séparer le socle du corps du patient ni modifier le réglage des arceaux, ce fourreau étant pourvu intérieurement d'un manchon (33) assurant l'immobilisation de la sonde à l'intérieur du porte-sonde.

3. Le support de sonde de la revendication 1, dans lequel le porte-sonde est de forme sensiblement cylindrique, de manière à autoriser une rotation axiale de la sonde sur elle-même, sans déplacement du socle ni des arceaux.

4. Le support de sonde de la revendication 1, dans lequel les moyens de blocage comprennent un écrou (40) disposé sur une partie filetée (38) du porte-sonde en saillie par rapport aux arceaux, le serrage de cet écrou assurant en un seul mouvement le blocage et l'immobilisation en position de l'ensemble arceaux/porte-sonde.

5. Le support de sonde de la revendication 1, dans lequel le porte-sonde est orientable à l'intérieur d'un cône de 90° d'angle au sommet, l'axe du cône étant perpendiculaire à la surface de contact.

## Patentansprüche

1. Halterung einer echographischen Sonde, insbesondere einer Sonde für die Echokardiographie mit
    – einem Sockel (10), der eine auf den zu untersuchenden Ort am Körper eines Patienten aufzulegende Kontaktoberfläche aufweist,
    – einem Sondenträger (30), welcher die Sonde (50) unbeweglich aufnimmt und mit Mitteln versehen ist, welche die Sonde axial gegen den Körper des Patienten drücken,
    – einer Haltevorrichtung für den Sondenträger am Sockel mit der Möglichkeit der Orientierung des Sondenträgers durch Schwenken um einen Punkt, welcher nächst der Kontaktoberfläche gelegen ist, und
    – einer Blockiervorrichtung (40) zum Blockieren des Sondenträgers in Position,
dadurch **gekennzeichnet**, daß
    – die Haltevorrichtung für den Sondenträger am Sockel (2) halbkreisförmige Bügel (20, 20') aufweist, welche um entsprechende, zueinander senkrechte Achsen (X,Y) schwenkbar sind, die zueinander senkrecht in einer gemeinsamen Ebene liegen, welche parallel zur Ebene des Sockels und in unmittelbarer Nähe der Kontaktoberfläche gelegen ist, derart, daß die Sonde um eine Drehmitte schwenken kann, welche praktisch mit dem Kontaktpunkt der Sonde mit dem Patienten zusammenfällt, wobei die Bügel je einen langgestreckten Schlitz (21, 21') aufweisen, welcher sich über den größten Teil der Bügellänge erstreckt, und
    – der Sondenträger im Schnittpunkt der beiden Schlitze der Bügel plaziert ist.

2. Halterung nach Anspruch 1, bei der der Sondenträger eine Hülse (32) aufweist, die nach oben zum Aufnehmen der Sonde offen ist, ohne daß dabei der Sockel vom Körper des Patienten getrennt noch die Einstellung der Bügel modifiziert zu werden braucht, wobei die Hülse innen eine Muffe (33) unterstützt, welche die Unbeweglichkeit der Sonde im Inneren des Sondenträgers gewährleistet.

3. Halterung nach Anspruch 1, bei der der Sondenträger im wesentlichen zylindrisch ausgebildet ist, derart, daß er eine axiale Drehung der Sonde um ihre Achse ohne Verlagerung des Sockels oder der Bügel zuläßt.

4. Halterung nach Anspruch 1, bei welcher die Blockiervorrichtung eine Mutter (40) aufweist, welche auf einem Gewindeabschnitt (38) des Sondenträgers die Bügel übergreifend angeordnet ist, wobei das Anziehen dieser Mutter in einer einzigen Bewegung das Blockieren und das Stillsetzen in Position der Bügel- /Sondenträger-Anordnung zur Folge hat.

5. Halterung nach Anspruch 1, bei welcher der Sondenträger im Inneren um einen Kegel mit einem 90°-Spitzenwinkel orientierbar ist, wobei die Achse des Kegels senkrecht auf der Kontaktfläche steht.

## Claims

1/ A support for an echographic transducer, in particular an echocardiographic transducer, the support comprising:
    a base (10) having a contact surface suitable for being pressed against the body of a patient at the position which is to be examined;
    a transducer carrier (30) removeably receiving the transducer (50) and provided with means for urging the transducer axially against the body of the patient;
    means for mounting the transducer carrier on the base with the possibility of pointing the transducer carrier by pivoting about a point situated in the proximity of the contact surface; and
    locking means (40) for locking the transducer carrier in position;
    characterized in that:
    the means for mounting the transducer carrier on the base comprise two semi-circular hoops (20, 20') pivotally mounted about respective mutually perpendicular axes (X, Y) extending in a common plane, which plane is parallel to the plane of the base and is situated in the proximity of the contact surface, each of said hoops having an elongate slot (21, 21') extending over the major portion of its length; and

the transducer carrier is placed in both hoop slots where they intersect.

2/ A transducer support according to claim 1, in which the transducer carrier comprises a sheath (32) which is open at its top end in order to receive the transducer without separating the base from the body of the patient and without changing the adjustment of the hoops, said sheath being provided with an inside sleeve (33) for fixing the transducer inside the transducer carrier.

3/ A transducer support according to claim 1, in which the transducer carrier is substantially cylindrical in shape, thereby enabling the transducer to rotate about its own axis without moving the base or the hoops.

4/ A transducer support according to claim 1, in which the locking means comprise a nut (40) disposed on a threaded portion (38) of the transducer carrier, which portion projects outside the hoops, with tightening of said nut providing a single locking movement for fixing the hoop and transducer carrier assembly in position.

5/ A transducer support according to claim 1, in which the transducer carrier is pointable within a cone having a 90° apex angle, with the axis of the cone being perpendicular to the contact surface.

## FIG_1

## FIG_2

## FIG_3